# EUROPEAN PATENT APPLICATION

(11) **EP 2 896 400 A1**
(43) Date of publication of application: **22.07.2015**
(21) Application number: 14151665.8
(22) Date of filing: 17.01.2014
(51) Int. Cl.: A61K 38/46, A61K 39/395, A61K 47/48, A61K 9/00, C07K 16/24, A61P 11/00

(54) **Method for increasing the bioavailability of inhaled compounds**

(71) Applicant: Université Catholique de Louvain, 1348 Louvain-La-Neuve (BE); Université de Liège, 4031 Angleur (Liege) (BE)
(72) Inventor: Vanbever, Rita, 1950 Kraainem (BE); Koussoroplis, Salomé, 1040 Etterbeek (BE); Cataldo, Didier, 4877 Olne (BE); Van Snick, Jacques, 1970 Wezembeek-Oppem (BE)
(74) Representative: Icosa

(57) **Abstract**

The present invention relates to a compound comprising one or more PEG moieties having a molecular weight of more than 12 kDa, wherein said compound is a therapeutic agent active for treating a respiratory disease. The present invention also relates to the use of a PEGylated therapeutic agent for treating a respiratory disease. Another object of the invention is a method for enhancing the bioavailability of a therapeutic agent, for enhancing the pulmonary residency of a therapeutic agent and/or for reducing the pulmonary clearance of a therapeutic agent, wherein said methods comprise the PEGylation of the therapeutic agent. In a preferred embodiment the respiratory disease is cystic fibrosis and the therapeutic agent dornase alpha or an anti-IL13 antibody.

## Description

### FIELD OF INVENTION

The present invention relates to the treatment of respiratory diseases with inhaled therapeutic compounds. In particular, the present invention relates to a method for enhancing the bioavailability of inhaled therapeutic compounds thereby enhancing their therapeutic efficacy, wherein said method comprises the PEGylation of said therapeutic compounds.

### BACKGROUND OF INVENTION

Although inhalation aerosols might offer a targeted therapy for respiratory diseases, the therapeutic efficacy of inhaled proteins is limited by the rapid clearance of macromolecules in the lungs. Indeed, inhaled proteins can be eliminated by mucociliary clearance. They can also be taken up by alveolar macrophages or metabolized in the pulmonary tissue and cross respiratory epithelia, finally being absorbed to some extent in the bloodstream. Accordingly, proteins, such as, for example, antibodies, have been shown to be mostly eliminated from the lungs within 24 hours (Lombry et al, 2004, Am J Physiol Lung Cell Mol Physiol 286: L1002-L1008), whereas after injection, the serum half-life of full-size antibodies, for example, may reach 21 days.

There is thus a need for methods for increasing bioavailability of inhaled therapeutic compounds.

In order to increase bioavailability of a therapeutic compound, additional ingredients to be concomitantly administered were described. For example, the international patent application WO2006/076277 describes compositions for increasing the bioavailability of pulmonary administered insulin, wherein said composition include EDTA. Moreover, the US patent application US2005/008633 discloses methods for enhancing pulmonary absorption and bioavailability of biologically active agents, wherein said methods comprise co-administering said agent with a macrophage inhibiting agent.

Specific formulations were also described in the art. For example, the international patent application WO2009/050726 describes the micronization of bupropion, a therapeutic agent, for improving its bioavailability after pulmonary administration. The US patent application US2010/087416 discloses aerosolized fluoroquinolones formulated with divalent or trivalent cations and having improved pulmonary availability for the treatment of bacterial infections of the lung and upper respiratory tract.

The modification of the therapeutic agent for increasing its bioavailability after pulmonary administration was also suggested in the art. For example, the French patent application FR 2 840810 describes the use of small peptides as vectors for enhancing the pulmonary bioavailability of a therapeutic compound. However, all experiments are carried out with injected therapeutic compounds. Consequently, FR 2 840810 does not provide any evidence that such a modified compound may resist to pulmonary clearance.

PEGylation of proteins has been described in the art. In particular, a few peptides and proteins conjugated to small PEGs have been delivered to the lungs in previous studies. However, PEGylation was used to protect the protein from local proteolysis and thereby increase the systemic absorption of the intact macromolecule (Youn et al, 2008, Journal of controlled release 125(1): 68-75), to improve the biocompatibility of toxic antimicrobial peptides (Morris et al, 2012, Antimicrobial agents & chemotherapy, 56(6): 3298-3308) or to increase the local activity of superoxide dismutase in hyperoxia-induced pulmonary injury (Tang et al, 1993, Journal of applied physiology 74(3): 1425-1431).

US2012/0071402 describes PEGylated insulin analogues exhibiting resistance towards proteases for pulmonary administration, whose proteases resistance is due to specific mutations. PEGylation is described has decreasing molecular flexibility and concomitantly reducing the fibrillation propensity and limiting or modifying the pH precipitation zone.

Moreover, WO94/20069 describes the pulmonary administration of a PEGylated protein comprising 6 kDa PEG moieties, and demonstrates that a protein to which a polyethylene glycol molecule has been attached may be absorbed by the lung into the blood stream. However, WO94/20069 discloses that PEGylated proteins are eliminated from serum and lung within 24 hours. Consequently, the reduced pulmonary bioavailability of these protein constructs limits their use for treating a pulmonary disease.

The current scientific view is that mucociliary clearance in the lungs will clear compounds that are unable to penetrate the mucus, that bind to mucin fibers or that freely diffuse through the mucus but are unable to cross the airway epithelium effectively. Therefore, binding to mucus has not been considered desirable for pulmonary drug delivery. Large PEG chains (≥10-12 kDa) confer mucoadhesion to nanoparticles (Wang et al, 2008, Angew Chem Int Ed Engl., 47(50): 9726-9729). The skilled artisan would thus have been taught away the use of large PEG chains for enhancing the pulmonary bioavailability of therapeutic agents.

On the contrary, the inventors herein surprisingly demonstrate that the coupling of large PEG chains to proteins sustains their presence within the lung over a few days. Therefore, the present invention relates to PEGylated therapeutic agents for treating pulmonary or respiratory diseases.

### SUMMARY

The present invention thus relates to a compound comprising one or more PEG moieties, wherein said compound is a therapeutic agent active for treating a respiratory disease, wherein the PEG moiety has a molecular weight of more than 12 kDa, provided that said therapeutic agent is not an anti-IL17 antibody or a fragment thereof.

In one embodiment, said compound is selected from peptides, polypeptides and proteins, preferably is selected from the group comprising inhibitors of cyotkines, inhibitors of adhesion molecules, inhibitors of proteases, antibodies and antibody fragments, cytokines, decoy cytokines, cytokine receptors, deoxyribonucleases, immunosuppressant drugs. In one embodiment, said therapeutic agent is dornase alpha.

In one embodiment, said respiratory disease is selected from inflammatory lung diseases, obstructive lung diseases, restrictive lung diseases, respiratory tract infections, malignant tumors, benign tumors, pleural cavity diseases, pulmonary vascular diseases, emphysema and pulmonary hyperplasia, preferably said respiratory disease is asthma or cystic fibrosis.

In one embodiment, the PEG moiety has a molecular weight of at least 20 kDa, preferably of at least 40 kDa. In one embodiment, the PEG moiety is branched or forked.

The present invention also relates to a PEGylated therapeutic agent for use in treating a respiratory disease, wherein said PEGylated therapeutic agent is to be administered by respiratory administration.

In one embodiment, said compound is selected from peptides, polypeptides and proteins, preferably is selected from the group comprising inhibitors of cyotkines, inhibitors of adhesion molecules, inhibitors of proteases, antibodies and antibody fragments, cytokines, decoy cytokines, cytokine receptors, deoxyribonucleases, immunosuppressant drugs.

In one embodiment, said respiratory disease is selected from inflammatory lung diseases, obstructive lung diseases, restrictive lung diseases, respiratory tract infections, malignant tumors, benign tumors, pleural cavity diseases, pulmonary vascular diseases, emphysema and pulmonary hyperplasia, preferably said respiratory disease is asthma or cystic fibrosis.

In one embodiment, the PEG moiety of the PEGylated therapeutic agent has a molecular weight of at least 12 kDa, preferably of at least 20 kDa, more preferably of at least 40 kDa. In one embodiment, the PEG moiety of the PEGylated therapeutic agent is linear, branched or forked.

Another object of the invention is a method for enhancing the bioavailability of a compound to be administered by respiratory administration, preferably by inhalation, wherein said method comprises attaching one or more PEG moieties on said compound.

The present invention also relates to a method for reducing the pulmonary clearance of a compound, wherein said method comprises attaching one or more PEG moieties to the compound.

Another object of the invention is a method for enhancing the pulmonary residency of a compound, wherein said method comprises attaching one or more PEG moieties to the compound. In one embodiment, the pulmonary residency of the pulmonary compound is of at least 24 hours, preferably of at least 36 or 48 hours.

### DEFINITIONS

In the present invention, the following terms have the following meanings:
- "**PEG**" or "**polyethylene glycol**" refers to any water soluble poly(ethylene glycol) or poly(ethylene oxide). The expression PEG thus comprises the structure (CH₂CH₂O)ₙ, wherein n is an integer from 2 to about 1000. A commonly used PEG is end-capped PEG, wherein one end of the PEG termini is end-capped with a relatively inactive group such as alkoxy, while the other end is a hydroxyl group that may be further modified by linker moieties. In one embodiment, the capping group is methoxy and the corresponding end-capped PEG is denoted mPEG. Hence, mPEG is CH₃O(CH₂CH₂O)ₙ, wherein n is an integer from 2 to about 1000. In another embodiment, the capping group is hydroxyl and the corresponding end-capped PEG is hydroxyPEG. "PEG" followed by a number (not being a subscript) indicates a PEG moiety with the approximate molecular weight equal the number multiplied by 1,000. Hence, "PEG40" is a PEG moiety having an approximate molecular weight of 40 kDa.
- "**Alkoxy**" refers to any O-alkyl or O-aryl group.
- **"PEGylation"** refers to the attachment of one or more PEG moieties to a compound, preferably the covalent attachment of one or more PEG moieties to therapeutic agent. In one embodiment, the PEG moiety may be attached by nucleophilic substitution (acylation) on N-terminal alpha-amino groups or on lysine residue(s) on the gamma-positions, e.g., with OSu-activated esters. In another embodiment, the PEG moiety may be attached by reductive alkylation on amino groups present in the therapeutic agent using PEG-aldehyde reagents and a reducing agent, such as sodium cyanoborohydride. In another embodiment, the PEG moiety may be attached to the sidechain of an unpaired cysteine residue in a Michael addition reaction using for example PEG maleimide reagents. Other PEGylation methods include, but are not limited to, bridging PEGylation, transglutaminase PEGylation, PEGylation using genetic engineering, releasable linkers PEGylation. For a review on PEGylation methods, see Pasut and Veronese, 2012, Journal of controlled release, 161:461-472 and Roberts et al., 2012, Advanced drug delivery reviews, 64:116-127. In one embodiment, the PEG moieties are attached to side chain(s) of lysine or cysteine residue(s) when present or attached to the N-terminal amino group(s) within the therapeutic compound.
- "**Linker**" refers to a chemical moiety which connects an -HN- group of the therapeutic agent with the -O- group of a PEG moiety. In a preferred embodiment, the linker does not have any influence on the desired action of the final PEGylated therapeutic agent, especially it does not have any adverse influence. The linker is typically a derivative of a carboxylic acid, wherein the carboxylic acid functionality is used for attachment to the therapeutic agent via an amide bond. Examples of linkers include, but are not limited to, an acetic acid moiety with the linking motif: CH₂CO, a propionic acid moiety with the linking motif: CH₂CH₂CO or CHCH₃CO, a butyric acid moiety with the linking motif: CH₂CH₂CH₂CO or CH₂CHCH₃CO, a CO group, N-(aminocarbonyl)succinimide derivatives (such as, for example, N-(N-propylpropanamide)succinimide, N-(N-propylhexanamide)succinimide and N-(N-ethylpropanamide)succinimide), pentanoic acid ((CH₂)₅C0), α-methyl butanoic acid (CH₂CH₂CH(CH₃)CO), succinic acid (CO(CH₂)₂CO), glutaric acid (CO(CH₂)₃CO), succinamide derivatives (such as, for example, (CH₂)₂NHCO(CH₂)₂CO), glutaramide derivatives (such as, for example, (CH₂)₃NHCO(CH₂)₃CO and (CH₂)₂NHCO(CH₂)₃CO). Preferably, the linker is (CH₂)₃NHCO(CH₂)₃CO.
- "**Bioavailability**" refers to the amount of the therapeutic agent that becomes available to the target tissue after administration. In the context of the present invention, the target tissue is preferably the lung, and the term "bioavailability" may specifically refer to "pulmonary bioavailability". Determination of bioavailability is well known in the art and can be calculated by measuring the Area Under the Curve (AUC) of a particular therapeutic agent concentrations within a biological fluid over a period of time. In one embodiment, the pulmonary bioavailability may be determined *in vivo* by detecting and measuring the amount of therapeutic agent within expectorations, non-induced or induced, or within bronchoalveolar lavage (BAL) after pulmonary administration of the PEGylated therapeutic agent of the invention. In another embodiment, the pulmonary bioavailability may be determined *in vitro* on a monolayer of respiratory cells by measuring the retention of the compounds on the apical side of the monolayer.
- "**Respiratory administration**" refers to the administration of therapeutic agent to the respiratory tract, such as, for example, by nasal administration, by inhalation or by insufflation.
- "**Protein**"**, "polypeptide**", "**peptide**"**:** As used herein, the term "peptide" refers to a short chain of amino acid monomers linked together by peptide bonds, while the term "polypeptide" refers to a linear polymer of amino acids (preferably at least 50 amino acids) linked together by peptide bonds. A protein specifically refers to a functional entity formed of one or more polypeptides, and optionally of non-polypeptides cofactors.
- "**Respiratory disease**" refers to all pathological conditions affecting the organs and tissues involved in gas exchange. Examples of respiratory diseases thus include, without limitation, diseases affecting the upper respiratory tract, the trachea, the bronchi, the bronchioles, the alveoli, the pleura and pleural cavity, as well as diseases affecting the nerves and muscles of breathing.
- "**Treating**" refers to both therapeutic treatment and prophylactic or preventative measures; wherein the object is to prevent or slow down (lessen) the targeted respiratory disease. Those in need of treatment include those already with the disease as well as those prone to have the disease or those in whom the disease is to be prevented. A subject is successfully "treated" for a respiratory disease if, after receiving a therapeutic amount of the PEGylated therapeutic agent of the present invention, the subject shows observable and/or measurable reduction in or absence of one or more of the following: improvement of respiratory function; reduction in the number of pathogenic cells; reduction in the percent of total cells that are pathogenic; and/or relief to some extent, of one or more of the symptoms associated with the specific respiratory disease; reduced morbidity and mortality, and improvement in quality of life issues. In one embodiment wherein the targeted respiratory disease is cystic fibrosis, the treated subject shows reduction in the occurrence frequency of respiratory infections. The above parameters for assessing successful treatment and improvement in the respiratory disease are readily measurable by routine procedures familiar to a physician. In one embodiment, the respiratory function may be assessed by FEV1 (forced expiratory volume in 1 second) or by DLCO (diffusing capacity of the lung for carbon monoxide).
- "**Therapeutically effective amount**" means level or amount of the PEGylated therapeutic agent of the invention that is aimed at, without causing significant negative or adverse side effects to the target, (1) delaying or preventing the onset of a respiratory disease; (2) slowing down or stopping the progression, aggravation, or deterioration of one or more symptoms of a respiratory disease; (3) bringing about ameliorations of the symptoms of a respiratory disease; (4) reducing the severity or incidence of a respiratory disease; or (5) curing a respiratory disease. A therapeutically effective amount may be administered prior to the onset of the respiratory disease, for a prophylactic or preventive action. Alternatively or additionally, the therapeutically effective amount may be administered after initiation of the respiratory disease, for a therapeutic action.
- "**About**" preceding a figure means plus or less 10% of the value of said figure.

### DETAILED DESCRIPTION

The inventors herein demonstrated that the coupling of PEG chains to proteins sustains their presence within the lung over a few days (see Examples). Without willing to be bound to a theory, the inventors suggest that mucoadhesion is an important mechanism underlying the retention of PEGylated proteins within the lungs, and hypothesize that PEGylated constructs are maintained in the airway lumen due to their binding and entanglement with the mucin fibers that form the mucus gel and with cell-surface mucins anchored in the cell membrane.

The present invention thus relates to PEGylated therapeutic agents, in particular to PEGylated therapeutic agents useful for treating, or for use in treating, a pulmonary disease.

In one embodiment, the therapeutic agent is a protein, a polypeptide or a peptide.

In one embodiment, the therapeutic agent is an inhibitor of a cytokine. In one embodiment, an inhibitor of a cytokine is selected from the group comprising an antibody directed to said cytokine, a soluble receptor of said cytokine and an antibody directed to the receptor of said cytokine.

In one embodiment, the therapeutic agent is an antibody or a fragment thereof.

The term "antibody" (Ab) as used herein includes monoclonal antibodies, polyclonal antibodies, multispecific antibodies (e.g., bispecific antibodies), and antibody fragments.

The basic four-chain antibody unit is a heterotetrameric glycoprotein composed of two identical light (L) chains and two identical heavy (H) chains. The L chain from any vertebrate species can be assigned to one of two clearly distinct types, called kappa and lambda, based on the amino acid sequences of their constant domains (CL). Depending on the amino acid sequence of the constant domain of their heavy chains (CH), immunoglobulins can be assigned to different classes or isotypes. There are five classes of immunoglobulins: IgA, IgD, IgE, IgG, and IgM, having heavy chains designated alpha, delta, epsilon, gamma and mu, respectively. The gamma and alpha classes are further divided into subclasses on the basis of relatively minor differences in CH sequence and function, e.g., humans express the following subclasses: IgG1, IgG2, IgG3, IgG4, IgA1, and IgA2. Each L chain is linked to an H chain by one covalent disulfide bond, while the two H chains are linked to each other by one or more disulfide bonds depending on the H chain isotype. Each H and L chain also has regularly spaced intrachain disulfide bridges. Each H chain has at the N-terminus, a variable domain (VH) followed by three constant domains (CH) for each of the alpha and gamma chains and four CH domains for mu and epsilon isotypes. Each L chain has at the N-terminus, a variable domain (VL) followed by a constant domain (CL) at its other end. The VL is aligned with the VH and the CL is aligned with the first constant domain of the heavy chain (CH1). Particular amino acid residues are believed to form an interface between the light chain and heavy chain variable domains. The pairing of a VH and VL together forms a single antigen-binding site. An IgM antibody consists of five of the basic heterotetramer units along with an additional polypeptide called a J chain, and therefore, contains ten antigen binding sites, while secreted IgA antibodies can polymerize to form polyvalent assemblages comprising 2-5 of the basic 4-chain units along with J chain. In the case of IgGs, the 4-chain unit is generally about 150,000 Daltons. For the structure and properties of the different classes of antibodies, see, e.g., Basic and Clinical Immunology, 8th edition, Daniel P. Stites, Abba I. Terr and Tristram G. Parslow (eds.), Appleton & Lange, Norwalk, Conn., 1994, page 71, and Chapter 6.

The term "monoclonal antibody" as used herein refers to an antibody obtained from a population of substantially homogeneous antibodies, i.e., the individual antibodies comprised in the population are identical except for possible naturally occurring mutations that may be present in minor amounts. Monoclonal antibodies are highly specific, being directed against a single antigenic site. Furthermore, in contrast to polyclonal antibody preparations that include different antibodies directed against different determinants (epitopes), each monoclonal antibody is directed against a single determinant on the antigen. In addition to their specificity, the monoclonal antibodies are advantageous in that they may be synthesized uncontaminated by other antibodies. A "chimeric" antibody refers to an antibody in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies (see U.S. Pat. No. 4,816,567; and Morrison et al., Proc. Natl. Acad. Sci. USA, 81:6851-6855 (1984)). In one embodiment, chimeric antibodies include antibodies having one or more non-human antigen binding sequences (e.g., CDRs) and containing one or more sequences derived from a human antibody, e.g., an FR or C region sequence. In addition, chimeric antibodies include those comprising a human variable domain antigen binding sequence of one antibody class or subclass and another sequence, e.g., FR or C region sequence, derived from another antibody class or subclass. Chimeric antibodies also include those derived from a different species, such as a non-human primate (e.g., Old World Monkey, Ape, etc). Chimeric antibodies also include primatized and humanized antibodies. Furthermore, chimeric antibodies may comprise residues that are not found in the recipient antibody or in the donor antibody. These modifications are made to further refine antibody performance. For further details, see Jones et al., Nature 321:522-525 (1986); Riechmann et al., Nature 332:323-329 (1988); and Presta, Curr. Op. Struct. Biol. 2:593-596 (1992).

An "antibody fragment" comprises a portion of an intact antibody, preferably the antigen binding or variable region of the intact antibody. Examples of antibody fragments include Fab, Fab', F(ab')2, and Fv fragments; diabodies; nanobodies (for a review, see Muyldermans, Annu. Rev. Biochem. 2013, 82:775-797), linear antibodies (see U.S. Pat. No. 5,641,870; Zapata et al., Protein Eng. 8(10): 1057-1062 [1995]); single-chain antibody molecules; and multispecific antibodies formed from antibody fragments. Papain digestion of antibodies produces two identical antigen-binding fragments, called "Fab" fragments, and a residual "Fc" fragment, a designation reflecting the ability to crystallize readily. The Fab fragment consists of an entire L chain along with the variable region domain of the H chain (VH), and the first constant domain of one heavy chain (CH1). Each Fab fragment is monovalent with respect to antigen binding, i.e., it has a single antigen-binding site. Pepsin treatment of an antibody yields a single large F(ab')2 fragment that roughly corresponds to two disulfide linked Fab fragments having divalent antigen-binding activity and is still capable of crosslinking antigen. Fab' fragments differ from Fab fragments by having additional few residues at the carboxy terminus of the CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments that have hinge cysteines between them. Other chemical couplings of antibody fragments are also known.

"Fv" is the minimum antibody fragment that contains a complete antigen-recognition and -binding site. This fragment consists of a dimer of one heavy- and one light-chain variable region domain in tight, non-covalent association. From the folding of these two domains emanate six hypervariable loops (three loops each from the H and L chain) that contribute the amino acid residues for antigen binding and confer antigen binding specificity to the antibody. However, even a single variable domain (or half of an Fv comprising only three CDRs specific for an antigen) has the ability to recognize and bind antigen, although at a lower affinity than the entire binding site.

"Single-chain Fv" also abbreviated as "sFv" are antibody fragments that comprise the VH and VL antibody domains connected into a single polypeptide chain. Preferably, the sFv polypeptide further comprises a polypeptide linker between the VH and VL domains that enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994); Borrebaeck 1995, infra.

The term "diabodies" refers to small antibody fragments prepared by constructing sFv fragments with short linkers (about 5-10 residues) between the VH and VL domains such that inter-chain but not intra-chain pairing of the V domains is achieved, resulting in a bivalent fragment, i.e., fragment having two antigen-binding sites. Bispecific diabodies are heterodimers of two "crossover" sFv fragments in which the VH and VL domains of the two antibodies are present on different polypeptide chains. Diabodies are described more fully in, for example, EP 404,097; WO 93/11161; and Hollinger et al., Proc. Natl. Acad. Sci. USA, 90:6444-6448 (1993).

In one embodiment, the therapeutic agent is an anti-interleukin-17A (IL17A) antibody or a fragment thereof, such as, for example, an anti-IL17A F(ab')₂ fragment. In another embodiment, the therapeutic agent is an anti-interleukin-13 (IL13) antibody or a fragment thereof, such as, for example, an anti-IL13 Fab' fragment. In another embodiment, the therapeutic agent is an antibody directed to, IL-4, IL-5, IL-9, IL-13, IL-17, IL-33, TNFα or a fragment thereof. A non-limiting example of an antibody directed to IL-5 is mepolizumab. Examples of antibodies directed to IL-13 include, but are not limited to, lebrikizumab and tralokinumab. A non-limiting example of an antibody directed to IL-9 is MEDI-528.

In another embodiment, the therapeutic agent is a cytokine receptor, preferably a soluble cytokine receptor, more preferably a receptor of a cytokine selected from the group comprising IL-13, IL-4, IL-5, IL-17, IL-9, IL-33 and TNFα A non-limiting example of a soluble receptor of TNFα is etanercept.

In another embodiment, the therapeutic agent is an antibody directed to a cytokine receptor or a fragment thereof, preferably an antibody directed to a receptor of a cytokine selected from the group comprising IL-13, IL-4, IL-5, IL-17, IL-9, IL-33, GM-CSF and TNFα Examples of antibodies or fragment thereof directed to TNFα receptor TNFR1 include, but are not limited to, GSK1995057 and GSK2862277. A non-limiting example of an antibody directed to the receptor of IL-4 is dupilumab. A non-limiting example of an antibody directed to the receptor of IL-5 is benralizumab. A non-limiting example of an antibody directed to the common beta chain receptor to IL-5 and GM-CSF.

In another embodiment, the therapeutic agent is an inhibitor of an adhesion molecule, such as, for example, ICAM1 or VCAM1. In one embodiment, an inhibitor of an adhesion molecule is an antibody directed to said adhesion molecule, a peptide or a small molecule.

In one embodiment, the therapeutic agent is an antibody directed to an adhesion molecule (such as, for example, ICAM1 or VCAM1) or to ligands thereof (such as, for example, LFA-1 and VLA-4).

In another embodiment, the therapeutic agent is a small molecule or a peptide inhibiting an adhesion molecule. Examples of small molecules or peptides inhibiting ICAM1 or VCAM1 are described in Yusuf-Makagiansar et al, Medical Research Reviews, 22(2): 146-167, 2002. Non-limiting examples of such small molecules or peptides include, but are not limited to, cyclic ICAM₁₁₋₂₁-derived peptides, peptides from both the alpha and beta-subunit of LFA-1, peptides containing residues 367-394 and Ala378 of ICAM1, cyclic peptides derived from IVAM1 and LCAM1, linear and cyclic peptides based on the LDV sequence, peptides containing the sequence ILDV, small molecule inhibitor based on the LDV sequence from CS1 FN (BIO-1494), CS1 peptide, glucocorticoids, NSAIDs, piroxicam, meloxicam, indomethacin, aceclofenac, diclofenac, salicylates, methotrexate, pentoxifylline, inhibitors of HMG coA reductase, p-arylthio cinnamides, BIRT-377 and the like.

In another embodiment, the therapeutic agent is an inhibitor of a protease (such as, for example, MMP9). In one embodiment, an inhibitor of a protease is an antibody directed to said protease, a peptide or a small molecule.

In one embodiment, the therapeutic agent is an antibody directed to a protease, such as, for example, an antibody directed to MMP9.

In another embodiment, the therapeutic agent is an inhibitor of a protease, such as, for example, an inhibitor of the serin protease (such as, for example, alpha1-antitrypsin).

In another embodiment, the therapeutic agent is a cytokine (such as, for example, interferon gamma 1b, interferon beta 1a, interleukin-2, GM-CSF and the like).

In another embodiment, the therapeutic agent is a decoy cytokine (such as, for example, a decoy form of IL-8).

In another embodiment, the therapeutic agent is a deoxyribonuclease (such as, for example, recombinant human deoxyribonuclease I).

In another embodiment, the therapeutic agent is an immunosuppressant drug, such as, for example, cyclosporine or basiliximab.

In another embodiment, the therapeutic agent is selected from the group comprising vasoactive intestinal peptide, glycan-binding decoy protein, ALX0171 nanobody.

In another embodiment, the therapeutic agent is an antibody directed to IgE, such as, for example, omalizumab or quilizumab.

In another embodiment, the therapeutic agent is an anti-M1 prime antibody, such as, for example, MEMP1972A.

In another embodiment, the therapeutic agent is an antibody directed to staph alpha toxin YTE.

In another embodiment, the therapeutic agent is an antibody directed to TSLP.

In one embodiment, said therapeutic agent is not an anti-interleukin-17A (IL17A) antibody or a fragment thereof, such as, for example, an anti-IL17A F(ab')₂ fragment.

In one embodiment, said therapeutic agent is dornase alpha.

In one embodiment, the therapeutic agent is active for treating a respiratory disease. Examples of respiratory diseases include, but are not limited to, inflammatory lung diseases, obstructive lung diseases, restrictive lung diseases, respiratory tract infections, malignant tumors, benign tumors, pleural cavity diseases, pulmonary vascular diseases, emphysema, pulmonary hyperplasia, bronchiectasis, atelectasis, lung abscess, occupational lung diseases, idiopathic interstitial lung diseases, pleurisy, hypersensitivity lung diseases, Goodpasture's syndrome, pulmonary alveolar proteinosis, pleura diseases, acute lung injury, and respiratory failure.

In another embodiment, the therapeutic agent is useful after lung transplantation, and may be active, for example, for treating or preventing lung graft rejection, graft versus host disease, and the like.

In another embodiment, the therapeutic agent is active for treating a respiratory condition related to the inhalation of a toxin, such as, for example, any toxin that may be used as a weapon and that induces toxicity when inhaled. In another embodiment, the therapeutic agent is active for treating a respiratory condition related to the inhalation of a spore, such as, for example, anthrax. A non-limiting example of a therapeutic agent useful for treating a respiratory condition related to the inhalation of anthrax is raxibacumab.

Examples of inflammatory lung diseases include, but are not limited to, asthma, cystic fibrosis, bronchiectasis, emphysema, chronic obstructive pulmonary disorder or acute respiratory distress syndrome.

Examples of obstructive lung diseases include, but are not limited to, chronic obstructive pulmonary disease (COPD), asthma, bronchitis, bronchiectasis, or bronchiolitis obliterable syndrome.

Examples of restrictive lung diseases include, but are not limited to, respiratory distress syndrome in infants and pulmonary fibrosis.

Examples of respiratory tract infections include, but are not limited to, upper respiratory tract infections (such as, for example, sinusitis, tonsillitis, otitis media, pharyngitis and laryngitis), lower respiratory tract infection (such as, for example, pneumonia, severe acute respiratory syndrome, pneumocystis pneumonia and the like).

Examples of malignant tumors include, but are not limited to, primary carcinomas of the lung, small cell lung cancer, non-small cell lung cancer (such as, for example, adenocarcinoma of the lung, squamous cell carcinoma of the lung, large cell lung carcinoma), other lung cancers (carcinoid, Kaposi's sarcoma, melanoma), lymphoma, head and neck cancer and pleural mesothelioma.

Examples of benign tumors include, but are not limited to, pulmonary hamartoma and congenital malformations (such as, for example, pulmonary sequestration and congenital cystic adenomatoid malformation).

Examples of pleural cavity diseases include, but are not limited to, pleural mesothelioma, pleural effusion and pneumothorax.

Examples of pulmonary vascular diseases include, but are not limited to, pulmonary embolism, pulmonary arterial hypertension, pulmonary edema, pulmonary hemorrhage.

Preferably, said respiratory disease is cystic fibrosis.

Examples of therapeutic agents active for treating cystic fibrosis include, but are not limited to, human deoxyribonuclease such as, for example, dornase alpha; antibodies and antibody constructs; alpha-1 anti-trypsine, interferon gamma 1b and the like.

Examples of therapeutic agents for treating asthma include, but are not limited to, antibodies and antibody constructs, such as, for example, antibodies or antibody constructs directed to a cytokine (such as, for example, IL-13, IL-4, IL-5, IL-17, IL-9, IL-33, TNFα) or a to cytokine receptor (such as, for example, receptors of IL-13, IL-4, IL-5, IL-17, IL-9, IL-33, TNFα), or to an adhesion molecule (such as, for example, ICAM1), or to a protease (such as, for example, MMP9).

Examples of therapeutic agents for treating emphysema include, but are not limited to, alpha-1 anti-trypsine.

In one embodiment of the invention, the PEGylated therapeutic agent comprises large PEG moieties, i.e. PEG moieties having a molecular weight of at least about 10 kDa, preferably of at least about 12 kDa, more preferably of at least about 13, 14, 15, 16, 17, 18, 19, 20, 25, 30, 40 kDa or more. In one embodiment, the PEG moiety has a molecular weight of more than about 12 kDa.

In one embodiment, the PEG moiety has a molecular weight ranging from about 10 kDa to about 60 kDa, preferably ranging from about 25 kDa to about 40 kDa.

Examples of PEG forms include branched, linear, forked (such as, for example, two armed or four armed PEG), dumbbell PEGs, and the like. Preferably, the PEG moiety of the invention is branched or forked PEG.

In one embodiment of the invention, the PEGylated therapeutic agent comprises one PEG moiety, or 2, 3, 4, 5, 6, 7, 8, 9, or 10 PEG moieties. In a preferred embodiment, the PEGylated therapeutic agent comprises only one PEG moiety.

In one embodiment, the one or more PEG moieties of the PEGylated therapeutic agent are not attached within the active site of the therapeutic agent, thereby preserving the activity of the therapeutic agent.

The present invention also relates to a composition comprising a PEGylated therapeutic agent as hereinabove described.

The present invention also relates to a pharmaceutical composition comprising a PEGylated therapeutic agent in association with at least one pharmaceutically acceptable excipient. As used herein, the term "pharmaceutically acceptable excipient" refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet non-pyrogenicity, general safety and purity standards as required by FDA Office of Biologics standards. In one embodiment, the pharmaceutical composition of the invention is sterile.

Another object of the invention is a medicament comprising a PEGylated therapeutic agent of the invention.

The present invention also relates to a PEGylated therapeutic agent of the invention, for treating, or for use in treating, a respiratory disease.

The present invention also relates to a method for treating a respiratory disease in a subject in need thereof, comprising administering to the subject a PEGylated therapeutic agent, preferably a therapeutically effective amount of a PEGylated therapeutic agent. In one embodiment of the invention, the PEGylated therapeutic agent is administered to the subject by respiratory administration, preferably by inhalation.

In one embodiment, the PEGylated therapeutic agent of the invention may be delivered by any of a variety of inhalation devices known in the art for administration of a therapeutic agent by inhalation. These devices include metered dose inhalers, nebulizers, dry powder inhalers, sprayers, and the like.

Some specific examples of commercially available inhalation devices suitable for the practice of this invention are Cyclohaler, Turbohaler™ (Astra), Rotahaler® (Glaxo), Diskus® (Glaxo), Spiros™ inhaler (Dura), devices marketed by Inhale Therapeutics, AERx™ (Aradigm), the Ultravent® nebulizer (Mallinckrodt), the Acorn II® nebulizer (Marquest Medical Products), the Ventolin® metered dose inhaler (Glaxo), the Spinhaler® powder inhaler (Fisons), or the like.

As those skilled in the art will recognize, the formulation of PEGylated therapeutic agent of the invention, the quantity of the formulation delivered and the duration of administration of a single dose depend on the type of inhalation device employed. For some aerosol delivery systems, such as nebulizers, the frequency of administration and length of time for which the system is activated will depend mainly on the concentration of PEGylated therapeutic agent in the aerosol. For example, shorter periods of administration can be used at higher concentrations of PEGylated therapeutic agent in the nebulizer solution. Devices such as metered dose inhalers can produce higher aerosol concentrations, and can be operated for shorter periods to deliver the desired amount of the PEGylated therapeutic agent. Devices such as powder inhalers deliver active agent until a given charge of agent is expelled from the device. In this type of inhaler, the amount of PEGylated therapeutic agent of the invention in a given quantity of the powder determines the dose delivered in a single administration.

In one embodiment, particles of the PEGylated therapeutic agent delivered by inhalation have a particle size preferably less than about 10 µm, more preferably in the range of about 1 µm to about 5 µm.

Advantageously for administration as a dry powder a PEGylated therapeutic agent is prepared in a particulate form with a particle size of less than about 10 µm, preferably about 1 to about 5 µm. Such formulations may be achieved by spray drying, milling, micronisation, or critical point condensation of a solution containing the PEGylated therapeutic agent of the invention and other desired ingredients.

Formulations of PEGylated therapeutic agent of the invention for administration from a dry powder inhaler typically include a finely divided dry powder containing the PEGylated therapeutic agent, but the powder can also include a bulking agent, carrier, excipient, another additive, or the like. Examples of additives include, but are not limited to, mono-, di-, and polysaccharides; sugar alcohols and other polyols, such as, e.g., lactose, glucose, raffinose, melezitose, lactitol, maltitol, trehalose, sucrose, mannitol, starch, or combinations thereof; surfactants, such as sorbitols, dipalmitoylphosphatidyl choline, or lecithin; or the like.

A spray including the PEGylated therapeutic agent of the invention can be produced by forcing a suspension or solution of the PEGylated therapeutic agent through a nozzle under pressure. The nozzle size and configuration, the applied pressure, and the liquid feed rate can be chosen to achieve the desired output and particle size. An electrospray can be produced, e.g., by an electric field in connection with a capillary or nozzle feed. Formulations of PEGylated therapeutic agent of the invention suitable for use with a sprayer will typically include the PEGylated therapeutic agent in an aqueous solution. The formulation may include agents such as an excipient, a buffer, an isotonicity agent, a preservative, a surfactant, and zinc. The formulation can also include an excipient or agent for stabilization of the PEGylated therapeutic agent, such as a buffer, a reducing agent, a bulk protein, or a carbohydrate. Examples of bulk proteins include, but are not limited to, albumin, protamine, or the like. Examples of carbohydrates include, but are not limited to sucrose, mannitol, lactose, trehalose, glucose, or the like. The PEGylated therapeutic agent formulation can also include a surfactant, which can reduce or prevent surface-induced aggregation of the PEGylated therapeutic agent caused by atomization of the solution in forming an aerosol. Various conventional surfactants can be employed, such as polyoxyethylene fatty acid esters and alcohols, and polyoxyethylene sorbitol fatty acid esters.

In an embodiment, the therapeutically effective amount of the PEGylated therapeutic agent of the invention may be appropriately determined in consideration of, for example, the age, weight, sex, difference in diseases, and severity of the condition of individual subject. It will be understood that the specific dose level and frequency of dosage for any particular subject may be varied and will depend upon a variety of factors including the activity of the PEGylated therapeutic agent employed, the metabolic stability and length of action of that PEGylated therapeutic agent, the age, body weight, general health, sex, diet, mode and time of administration, rate of excretion, drug combination, the severity of the particular condition, and the host undergoing therapy.

The present invention also relates to a method for enhancing the bioavailability, preferably the pulmonary bioavailability, of a therapeutic agent, wherein said method comprises the PEGylation of the therapeutic agent. In one embodiment, the therapeutic agent is active for treating a pulmonary disease. According to the invention, attaching one or more PEG moieties to the therapeutic agent enhances the bioavailability of said therapeutic agent, thereby enhancing the therapeutic efficacy of said therapeutic agent.

The present invention also relates to a method for reducing the pulmonary clearance of a therapeutic agent, thereby enhancing the pulmonary residency of said therapeutic agent, wherein said method comprises attaching one or more PEG moieties to the therapeutic agent.

In one embodiment, the pulmonary residency of the PEGylated therapeutic agent of the invention is of at least about 24 hours, preferably of at least about 36 or 48 hours.

In one embodiment, the amount of PEGylated therapeutic agent still present within the lung 24 hours post-delivery is of at least 20%, 30%, 40%, 50%, 60%, 70% or more.

In one embodiment, the amount of PEGylated therapeutic agent still present within the lung 48 hours post-delivery is of at least 10%, 15%, 20%, 25%, 30%, 35%, 40% or more.

In one embodiment, the amount of PEGylated therapeutic agent present within the lung is halved in at least 12 hours, preferably at least about 12, 16, 20, 24, 28, 30, 32, 33 hours or more.

In one embodiment, the subject is affected by, preferably is diagnosed with, a respiratory disease. In another embodiment, the subject is at risk of developing a respiratory disease. Examples of risk factors include, but are not limited to, predisposition to a respiratory disease, such as, for example, familial or genetic predisposition (such as, for example, mutation in the gene of the protein cystic fibrosis transmembrane conductance regulator (CFTR)); environmental conditions (such as, for example, atmospheric pollution), or lifestyle (such as, for example, smoking tobacco).

### BRIEF DESCRIPTION OF THE DRAWINGS

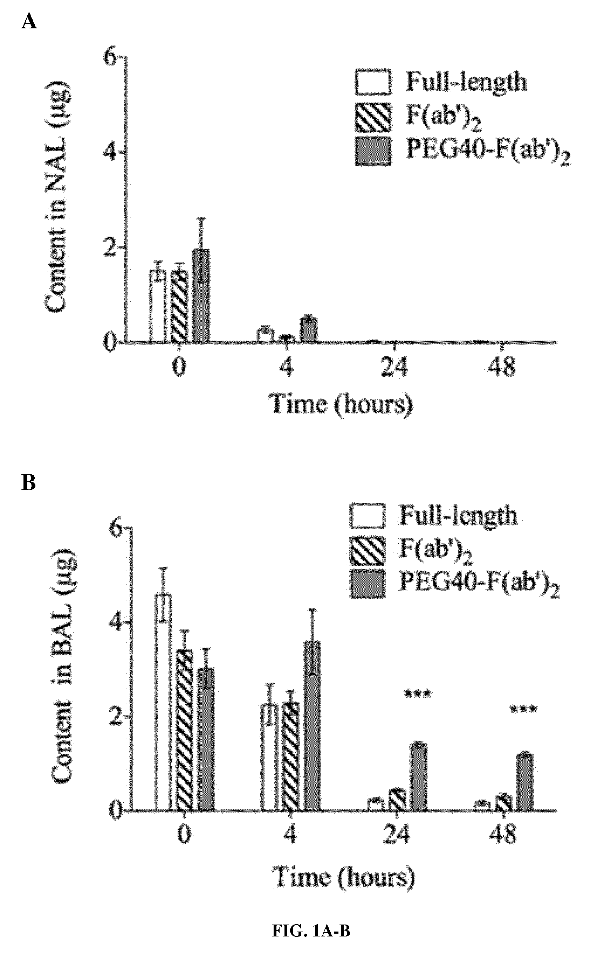

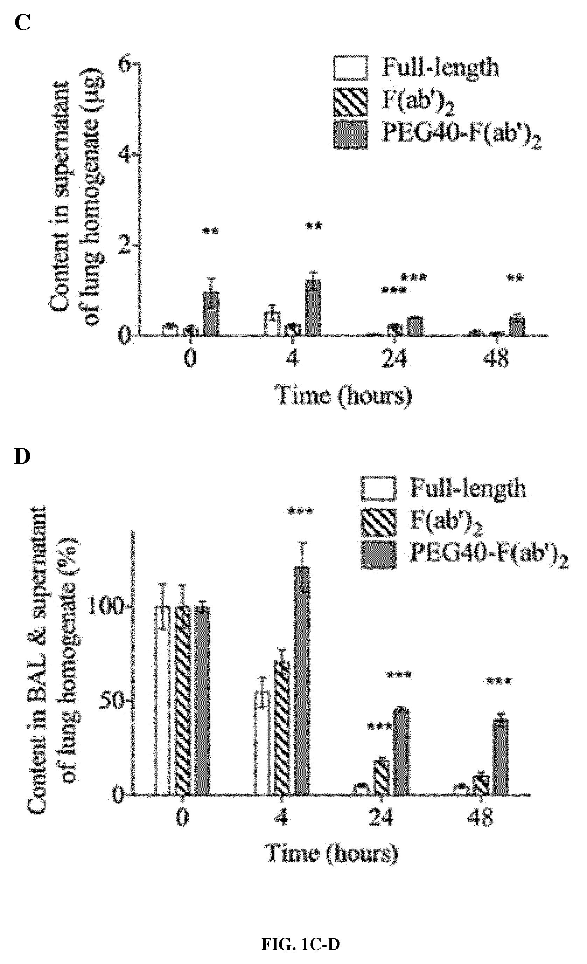

**Figure 1** is a combination of histograms showing the quantities of anti-IL-17A antibody constructs recovered from the respiratory tract 0, 4, 24 and 48 hours following intranasal delivery. (A-C) Amounts of antibody constructs recovered from (A) nasal lavage (NAL), (B) bronchoalveolar lavage (BAL) and (C) supernatant of lung homogenate, respectively, expressed in µg of protein. (D) The amount of antibody constructs recovered from the lungs, expressed as a percentage of the respective average amount recovered at time zero. Triton was used for the recovery of the PEGylated construct. The groups were compared to the full-length antibody at each time point. The data represent the mean values (± SEM) of ≥ three mice.

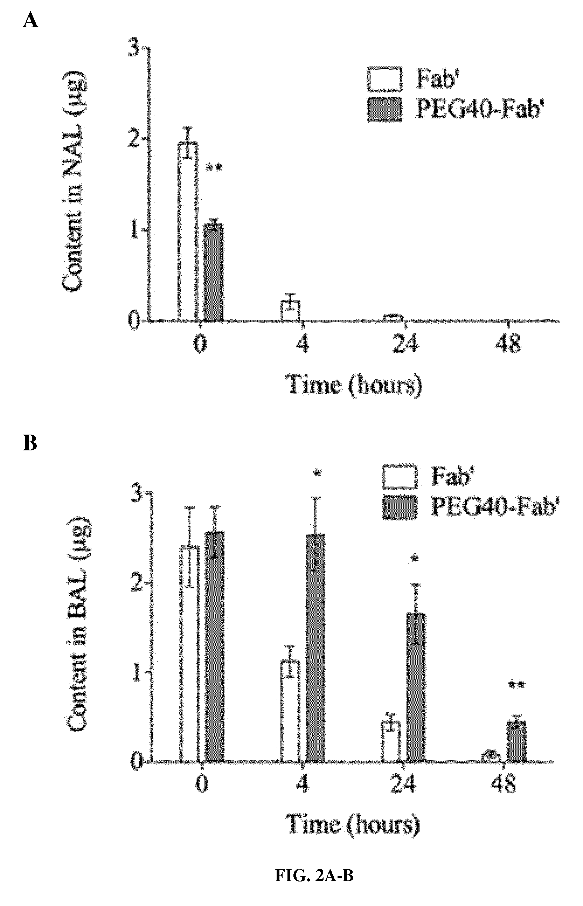

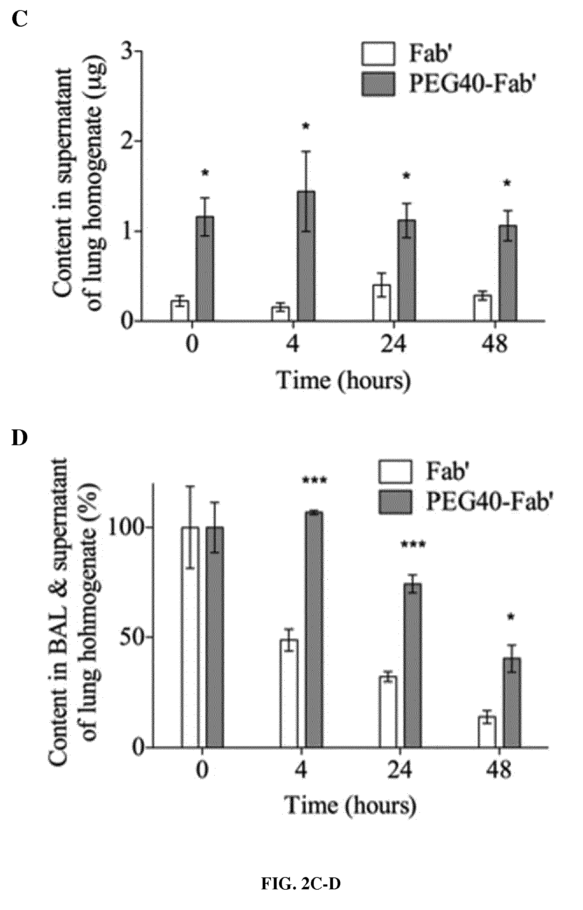

**Figure 2** is a combination of histograms showing the quantities of anti-IL-13 antibody constructs recovered from the respiratory tract 0, 4, 24 and 48 hours following intranasal delivery. (A-C) Amounts of antibody constructs recovered from (A) nasal lavage (NAL), (B) bronchoalveolar lavage (BAL) and (C) supernatant of lung homogenate, respectively, expressed in µg of protein. (D) Amount of antibody constructs recovered from the lungs, expressed as a percentage of the respective average amount recovered at time zero. Triton was used for the recovery of the PEGylated construct. The PEGylated construct was compared to the Fab' antibody fragment at each time point. The data represent the mean values (± SEM) of three mice.

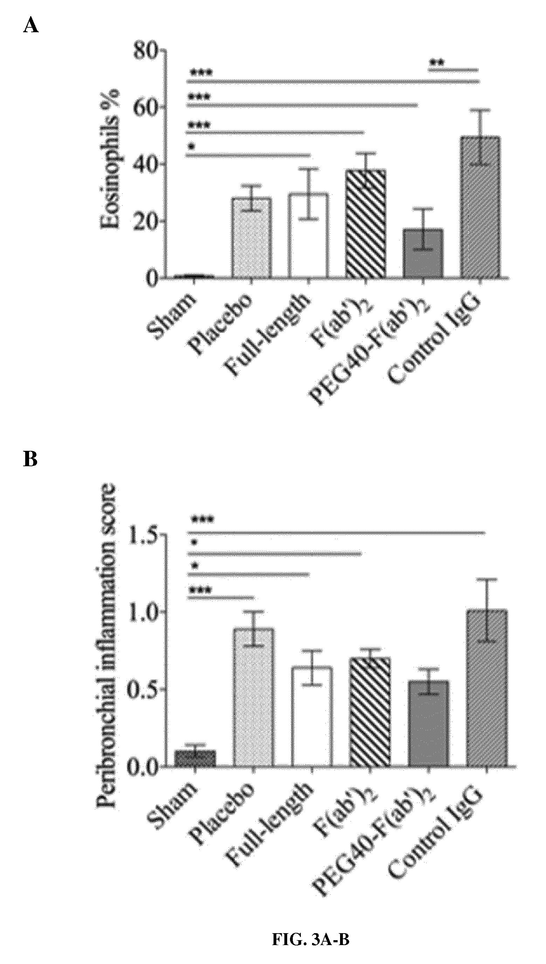

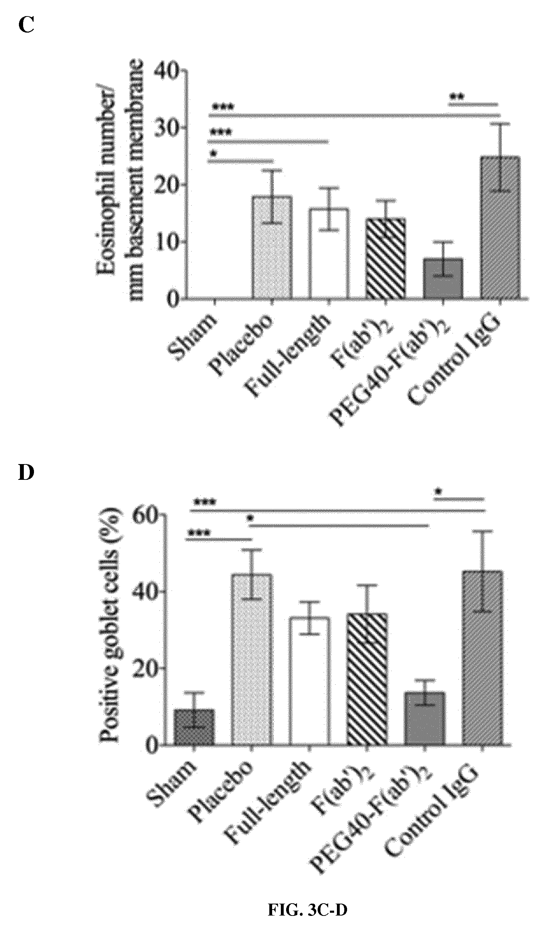

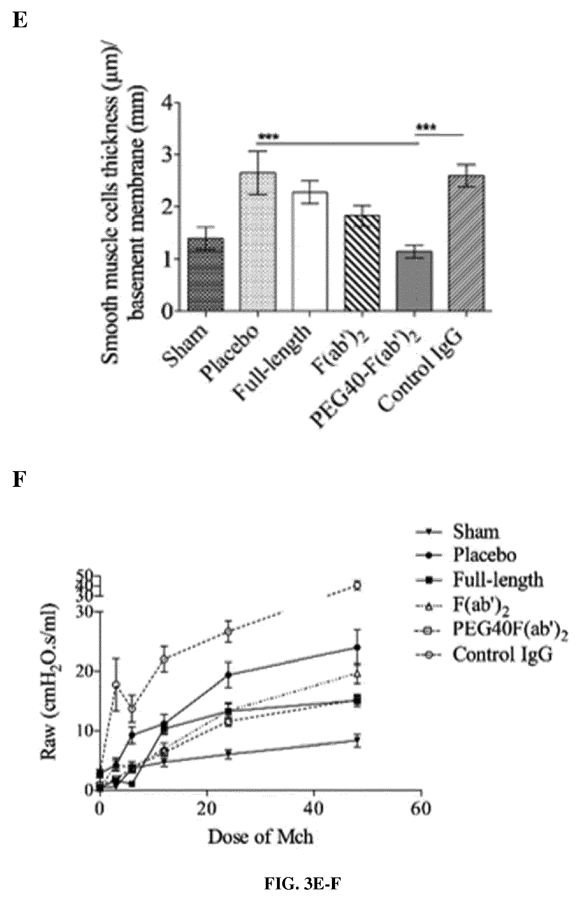

**Figure 3** is a combination of histograms showing the assessment of airway inflammation and hyperresponsiveness in a HDM-induced lung inflammation model following the delivery of the anti-IL-17A antibody constructs. (A) Eosinophils % in bronchoalveolar lavage; (B) peribronchial inflammation; (C) eosinophilic inflammation; (D) positive goblet cells; (E) smooth muscle cell thickness. Significant differences between groups are shown. (F) Responses to methacholine (Mhc). p < 0.001 for PEG40-F(ab')₂ and full-length antibody versus placebo and control IgG. The data represent the mean values (± SEM) of eight mice. Similar results were obtained in two independent experiments.

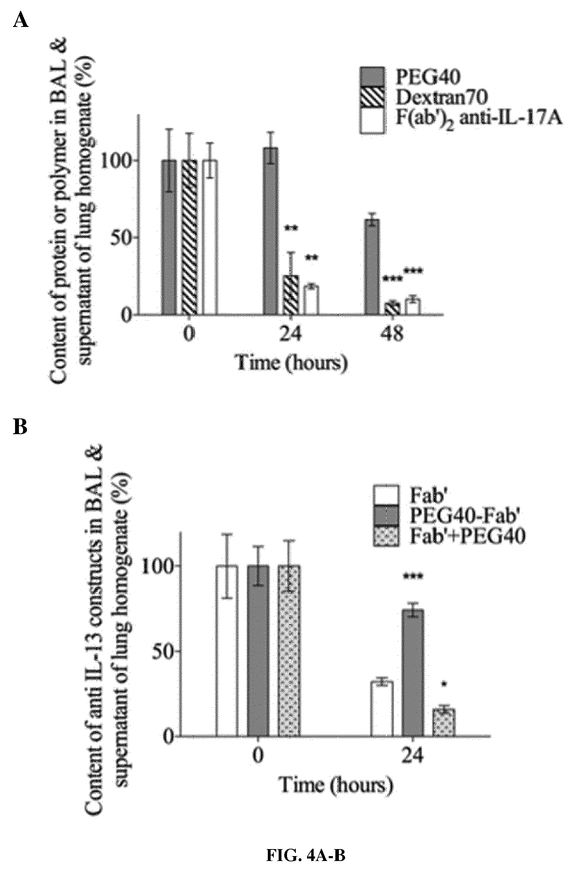

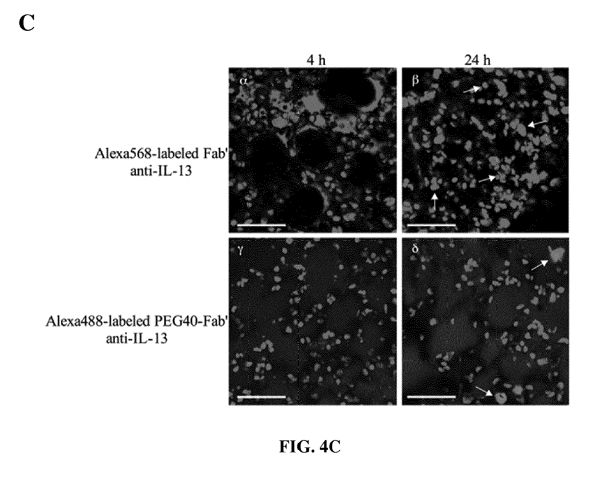

**Figure 4** is a combination of graphs and microscopy images showing the fate of anti-IL-17A F(ab')₂, anti-IL-13 Fab', PEG40 or dextran70 0, 24 and 48 hours following intranasal delivery. (A) PEG40, dextran70 and F(ab')₂ anti-IL-17A recovered from the lungs, expressed as a percentage of the respective average amount recovered at time zero. PEG40 was compared to the other groups at each time point. (B) Fab' anti-IL-13, PEG40-Fab' and a mixture of Fab' with PEG40, expressed as a percentage of the respective average amount recovered from the lungs at time zero. The Fab' was compared to the other groups at each time point. Triton was used for the recovery of the PEGylated construct or PEG40. The data represent the mean values (± SEM) of three mice. (C) Localization of Alexa568-labeled Fab' and Alexa488-labeled PEG40-Fab' anti-IL-13 antibody fragment in mouse lungs by confocal laser scanning microscopy. The alveoli were visualized (α) 4 and (β) 24 hours following delivery of Alexa568-labeled Fab' or (γ, δ) Alexa488-labeled PEG40-Fab' to the lungs. The lung tissue was colored in blue with nuclear stain DRAQ5™, and the arrows indicate alveolar macrophages loading fluorescent antibody constructs. The scale bars represent 50 µm.

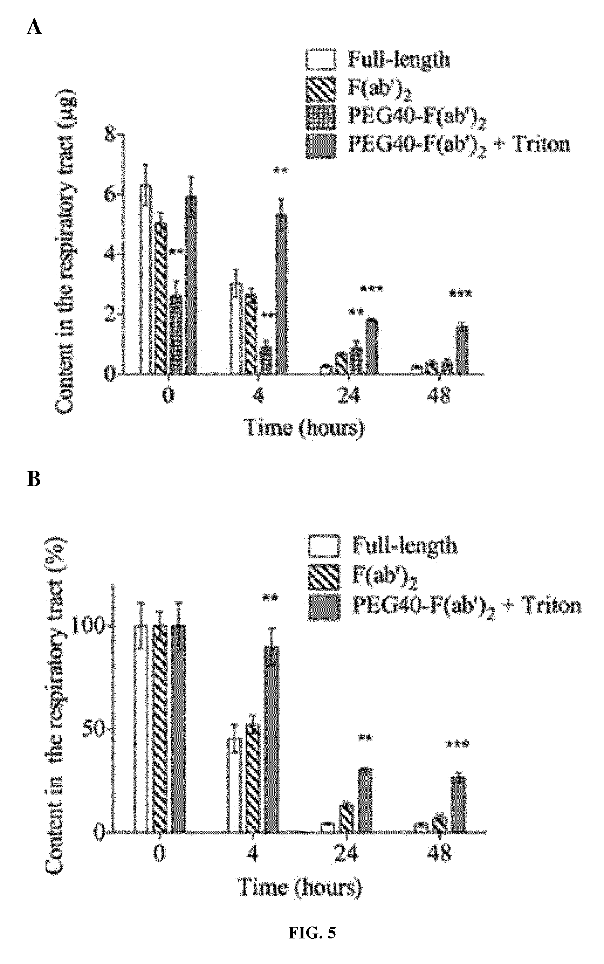

**Figure 5** is the combination of two histograms showing the quantities of anti-IL-17A antibody constructs recovered from the respiratory tract 0, 4, 24 and 48 hours following intranasal delivery. (A) Amount of antibody constructs recovered from the whole respiratory tract, expressed in µg of protein. The PEGylated antibody fragment was recovered with or without Triton. (B) Amount of antibody constructs recovered from the whole respiratory tract, expressed as a percentage of the respective average amount recovered at time zero. The groups were compared to the full-length antibody at each time point. The data represent the mean values (± SEM) of three mice.

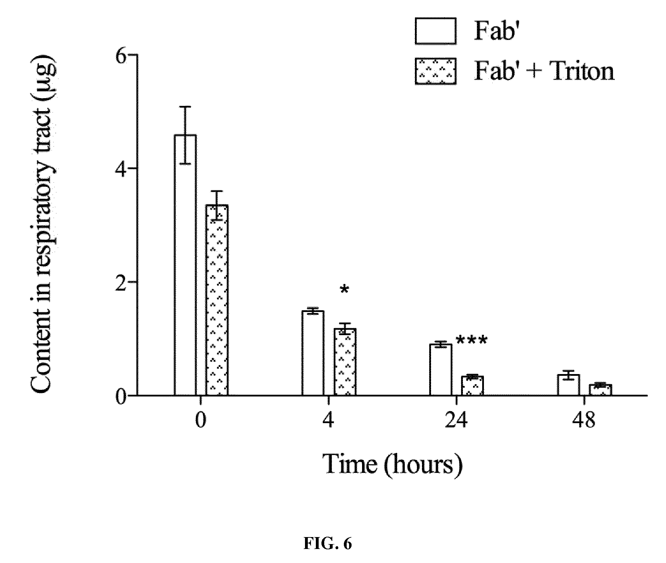

**Figure 6** is a histogram showing the effect of Triton on anti-IL-13 Fab' quantities recovered from the respiratory tract 0, 4, 24 and 48 hours following the intranasal delivery, expressed in µg of protein. Amount of Fab' recovered using Triton was compared to Fab' recovered without triton at each time point. The data represent the mean values (± SEM) of three mice.

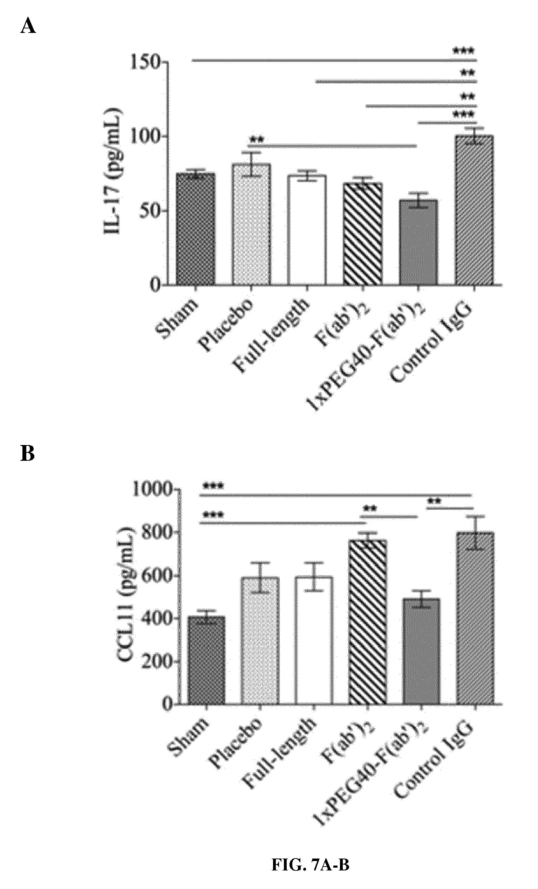

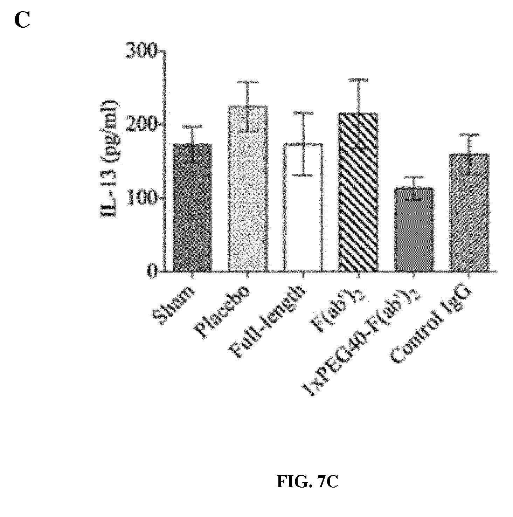

**Figure 7** is a combination of histograms showing key cytokines and chimiokine levels in a HDM-induced lung inflammation model following the delivery of the anti-IL-17A antibody constructs. Levels of IL-17 (A), CCL-11 (B) and IL-13 (C). Significant differences between groups are shown. The data represent the mean values (± SEM) of eight mice.

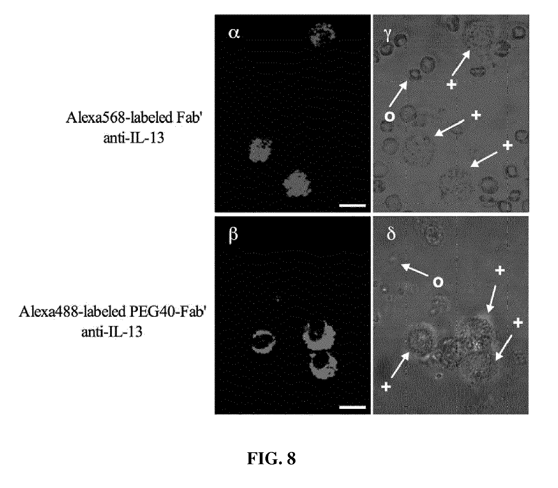

**Figure 8** is a microscopy image showing the visualization of the uptake of anti-IL-13 antibodies by alveolar macrophages using confocal laser scanning microscopy. Alveolar macrophages recovered by bronchoalveolar lavage 24 hours after delivery of (α) Alexa568-labeled Fab' or (β) Alexa488-labeled PEG40-Fab' to the respiratory tract.
The corresponding light field images are presented in (γ) and (δ) to visualize the alveolar macrophages (+) versus smaller red blood cells (o). The scale bars represent 50 µm.

### EXAMPLES

The present invention is further illustrated by the following examples.

### Materials and Methods

### Anti-IL-17A and Anti-IL-13 Antibodies

The murine anti IL-17A antibody was initially digested by pepsin to produce the F(ab')₂, which was conjugated to one molecule of two-armed 40 kDa PEG (abbreviated as PEG40-F(ab')₂) (Koussoroplis et al, 2013, International journal of pharmaceutics 454(1): 107-115). Briefly, anti-IL-17A hybridoma (MM17F3, IgG1-kappa) was derived from mice vaccinated with mouse IL-17A conjugated to ovalbumin (Uyttenhove et al, Eur. J. Immunol. 2006, 36: 2868-2874). Hybridoma cells were cultured in hybridoma serum free medium (HSFM; Invitrogen, Carlsbad, CA, USA) supplemented with IL-6 (1 ng/ml). The antibody was purified by passage over a Protein G Sepharose™ 4 Fast Flow column (GE Healthcare Bio-Sciences AB, Uppsala, Sweden) and eluted with 0.1 M glycine-HCl buffer pH 2.8. Eluted antibody was collected in tubes containing 1M Tris-HCl buffer pH 8 for immediate neutralization. Lipopolysaccharide (LPS) traces were removed by passage over Sartobind® IEC MA 15 (Sartorius-stedium biotech GmbH, Goettingen, Germany). Purified antibody was concentrated and dialyzed against phosphate buffered saline (PBS) before use. Murine Fab' and mono-PEGylated Fab' with one chain of 40 kDa PEG (abbreviated as PEG40-Fab') anti-IL-13 antibody fragments were provided by UCB Pharma.

### Kinetics of In Vivo Disposition in the Respiratory Tract

NMRI mice (6 to 9 week-old; Elevage Janvier, Le Genest-St-Isle, France) were anaesthetized using ketamine/xylazine (90/10 mg/kg) intraperitoneal injection. Biotinylated full-length, F(ab')₂ or PEG40-F(ab')₂ anti-IL-17A (10 µg of protein in 50 µl phosphate-buffered saline, PBS) was then administered intranasally. During the administration, the mouse was maintained in an upright position and 25 µl of the antibody solution was delivered drop by drop to each nostril using a micropipette in a way that it was inhaled naturally. Biotin labeling of the antibodies was performed using EZ-Link Sulfo-NHS-LC-Biotin reagent (Thermo Fisher Scientific, Rockford, IL, USA). At various pre-determined times (0, 4, 24 or 48 h) following the antibody administration, the mice were killed by a lethal injection of pentobarbital. A nasal lavage (NAL) was performed by cannulating the trachea towards the nasal cavity and instilling 3 ml Hanks' balanced salt solution (HBSS). The fluid emerging from the nostrils was collected. A BAL was then performed. One ml of HBSS was injected into the trachea, left for 30 s, followed by withdrawal and re-injection of 0.5 ml of the fluid and then all the BAL liquid was removed from the lungs. This procedure was repeated twice until a total volume of 3 ml was injected. Afterwards, the lungs were removed and ground to release the antibodies in 2 ml of HBSS with a tissue grinder Potter (Merck Erolab, Leuven, Belgium) for 2 min and the tissue grinder was rinsed with 1 ml of HBSS. For the PEGylated species, lavages and tissue processing was carried out using Triton® X-100 (Merck Millipore, Darmstadt, Germany) diluted at 1:1000 in HBSS. Blood, NAL, BAL and tissue homogenate samples were then centrifuged at 3000 g at 4 °C for 10 min. The supernatants were stored at -20 °C until they were assayed for antibody content by custom ELISA.

A similar protocol was used to study the fate of (a) Fab' and PEG40-Fab' anti-IL-13; (b) a mixture of PEG40-Fab' and unconjugated, two-armed 40-kDa PEG (in the same molar amount as the protein; NOF Corporation, Tokyo, Japan), and (c) 40 kDa methoxyl PEG Rhodamine B (PEG40; Nanocs, Boston, MA, USA) and 70 kDa Rhodamine B isothiocyanate-dextran (dextran70; Sigma-Aldrich, St. Louis, MO, USA). For (a) and (b), 10 µg of protein was intranasally administered to mice and antibody content was measured in samples by custom ELISA. For (c) 2.1 nmol was delivered and the content of fluorescent polymers in samples was measured using a Spectramax M3 microplate (Molecular devices, Orleans Drive, Sunnyvale, CA, USA).

The experimental protocols were approved by the Institutional Animal Care and Use Committee of the Université catholique de Louvain (Permit number: 2011-2/UCL/MD/028P). All studies were performed under anesthesia and all efforts were made to minimize suffering of the animals.

### Evaluation of Anti-IL-17A Constructs in a HDM-induced lung inflammation model

On days 0, 7 and 14, male Balb/c mice (8-week old, Elevage Janvier) were challenged intranasally with 100 µg HDM (Greer Laboratories, Lenoir, NC). On days 7, 10, 13 and 16, the antibody constructs were intranasally administered at a dose of 30 µg/administration for full-length anti-IL-17A before allergen challenge. The unconjugated anti-IL-17A F(ab')₂ fragment and the PEG40-F(ab')₂ were delivered in the same molar amount as the full-length anti-IL-17A (200 pmol/administration). "Sham mice" were mice that were only treated with PBS solution. The placebo group comprised allergen-challenged mice treated with PBS. On day 17, the mice were sacrificed after airway hyperresponsiveness had been measured.

To assess airway hyperresponsiveness, a 20 gauge polyethylene catheter was inserted into the exposed trachea of anesthetized mice and connected to a FlexiVent small-animal ventilator® (Scireq, Montreal, Canada).

After sacrifice, a cannula was inserted into the trachea of the mouse to rinse the lungs with PBS-EDTA 0.05 mM. The recovered BAL fluid was centrifuged, and the supernatants were stored at -80°C for further analyses while the cell pellets were resuspended in 1 ml PBS-EDTA 0.05 mM to carry out differential cell counts, which was performed by a skilled observer blinded to experimental details, based on morphological criteria. Cells were centrifuged on a slide and stained with Diff Quick® (Dade, Brussels, Belgium). A total of 300 cells were counted and eosinophil percentage was assessed. After BAL, the thorax was opened and the right lungs were excised and snap frozen in liquid nitrogen for protein extraction. The left lung was insufflated with 4% paraformaldehyde at a constant pressure and then embedded in paraffin for histological analyses. A peribronchial inflammation score was adjudged on each hematoxylin-eosin-stained slide. The score 0 was assigned for bronchi with no inflammation; score 1 corresponded to occasional mononuclear cells around bronchi, score 2 was assigned if there were from 1 to 5 layer(s) of inflammatory cells around bronchi. Six bronchi per mice were counted. Congo Red staining was performed on the lung sections to detect eosinophilic infiltration in the bronchial walls. Eosinophil counts were determined on 6 bronchi/ mouse and reported to the basal membrane epithelium perimeter measured with ImageJ Program. Alcian blue staining was also performed on the lung sections to detect goblet cells. Glandular hyperplasia was calculated as percentage of positive cells per total epithelial cells in randomly selected bronchi. Immunohistochemistry using an antibody against alpha-smooth muscle actin was performed to estimate the thickness of the smooth muscle cell layer around the bronchi.

Total protein extracts were prepared by incubating crushed lung tissue in a 2 M urea solution. Tissue lysates were centrifuged at 16,100 g for 15 min. The concentrations of interleukin-17, IL-13 and CCL11 were analyzed in the lung protein extracts using the R&D Duoset® Elisa Development kit (R&D Systems, Minneapolis, MN, USA).

The experimental procedures were approved by the Institutional Animal Care and Use Committee of the University of Liège (LA 1610002). All studies were performed under anesthesia and all efforts were made to minimize suffering of the animals

### Confocal Imaging of Lungs following the Delivery of PEGylated versus non-PEGylated Fragments

Fluorescent Fab' and PEG40-Fab' anti-IL-13 antibody constructs were administered intranasally at a dose of 0.3 nmol to NMRI mice. Labeling of Fab' and PEG40-Fab' with fluorescent dyes was carried out using protein labeling kits Alexa Fluor®568 and Alexa Fluor®488, respectively (Invitrogen, Invitrogen, Carlsbad, CA, USA). Zero, 4 or 24 hours following administration, the mice were anesthetized and an abdominal incision was made. The posterior vena cava was then cannulated with a BD Insyte-W™ catheter (Becton Dickinson Infusion Therapy Systems, Sandy, UT, USA) connected via a BD Connecta (Becton Dickinson Infusion Therapy Systems, Sandy, UT, USA) to two reservoirs containing: (i) 0.9% (w/v) NaCl and (ii) a fixative solution 4% (v/v) formaldehyde in 0.9% (w/v) NaCl. Both the carotids and jugulars were cut and solution (i) was perfused via the vasculature at a flow rate of 2 ml/min during 5 min. Then, lung fixation through the pulmonary vasculature was carried out using solution (ii) at a flow rate of 1 ml/min for 10 min. Subsequently, the thoracic cavity was opened and the lungs were removed. Slices of approximately 2 mm of lung lobes were immersed for 1 min in Draq5™ (Abcam, Cambridge, UK), diluted 1:100 (50 nM) in solution (ii). Then, the slices were briefly immersed in PBS and finally placed into a receptacle (Lab-Tek II chambered coverglass W/cover #1.5 borosilicate sterile; Lab-Tek® Brand products, Rochestern NY, USA) for analysis by confocal laser scanning microscopy. Each experimental condition was repeated at least twice.

Preparations were examined with an LSM 510 microscope (Zeiss, Jena, Germany) using a Plan-Apochromat 20x/0.8 objective (Zeiss, Jena, Germany). Fluorescent emissions from Alexa488-labeled PEG40-Fab', Alexa568-labeled Fab' and DRAQ5 were sequentially recorded in the green, then in the far-red, then red channels.

To evaluate the autofluorescent properties of the pulmonary tissue, samples were analyzed with confocal laser scanning microscopy without the presence of Alexa488-labeled PEG40-Fab'and Alexa568-labeled Fab'. The autofluorescence in both the green and red channels was found to be very low.

The uptake of antibodies by alveolar macrophages was further visualized by analyzing alveolar macrophages collected by BAL. The mice were killed by an overdose of pentobarbital 4 and 24 hours after intranasal delivery of fluorescent antibodies. The airways and lungs were washed with HBSS. The BAL was centrifuged at 700 g, 4°C for 10 min. The supernatant was removed and the cells were resuspended in 100 µl of HBSS. A few droplets of the cells suspension were directly placed into a sample holder to be analyzed by confocal laser scanning microscopy.

The experimental protocols were approved by the Institutional Animal Care and Use Committee of the Université catholique de Louvain (Permit number: 2011-2/UCL/MD/028P). All studies were performed under anesthesia and all efforts were made to minimize suffering of the animals.

### Results

### Kinetics of In Vivo Disposition in the Respiratory Tract

To study the impact of protein PEGylation on the pharmacokinetics of a protein within the lungs, we delivered the anti-IL-17A F(ab')₂ fragment conjugated to a two-armed 40-kDa PEG (PEG40-F(ab')₂), the unconjugated anti-IL-17A F(ab')₂ fragment and the full-length anti-IL-17A antibody to the respiratory tract in mice. We then measured the protein content of these fragments in nasal lavage, broncho-alveolar lavage (BAL) and lung homogenate at different times after delivery. The full-length antibody (150 kDa) and the unconjugated fragment (98 kDa) were mostly cleared from the respiratory tract within 24 hours (**Fig. 5A**)**.** Four hours post-delivery, the content of the unconjugated proteins in the respiratory tract had decreased to 60% of the initial dose that was deposited. Twenty-four hours post-delivery, the protein content further decreased to 5% and 18% of the dose initially deposited for the full-length antibody and F(ab')₂ fragment, respectively (**Fig. 5B**). The PEGylated anti-IL-17A F(ab')₂ (140 kDa) was recovered to a smaller extent from the lungs than the unconjugates, with half of the protein quantities recovered immediately after delivery and very small quantities at later time points (**Fig. 5A**)**.** We hypothesized that the low recovery of the PEG40-F(ab')₂ from the lungs could result from the binding of this fragment to the mucus covering the respiratory epithelia. Therefore, to increase recovery, we added Triton, a detergent able to dismantle the mucus gel, to the lavage solution. In the presence of Triton, the recovery of PEG40-F(ab')₂ at time zero increased to the levels of the unconjugated proteins (**Fig. 5A**)**.** Thereafter, the PEGylated fragment was very slowly cleared from the respiratory tract, with no significant clearance four hours post-delivery and with 27% of the dose initially deposited at time zero still present 48 hours post-delivery (**Fig. 5B**). In contrast to PEGylated proteins, the use of Triton for the non-PEGylated proteins did not increase recovery (**Fig. 6**).

**Figs. 1A** to **1D** present the detailed data on the content of the antibody constructs within the nasal lavage, BAL and lung tissue measured at different time points. The purpose of these measurements was to study potential antibody retention in the different parts of the respiratory tract. The nasal cavities did not retain any of the antibody constructs for more than a few hours (**Fig. 1A**). The lungs retained antibody constructs better than the nasal cavities, especially the PEGylated anti-IL-17A F(ab')₂ (**Figs. 1B & C**)**.** The PEG40-F(ab')₂ was mainly found in BAL, and one-fourth of the amount recovered from the lungs was found in the supernatant of lung homogenate at all sampling times. The unconjugated anti-IL-17A fragment and the full-length anti-IL-17A antibody were mainly found in BAL immediately after delivery and four hours later, whereas less than one-tenth of administered proteins was recovered from the supernatant of lung homogenate (**Figs. 1B & C**)**.** However, the fraction in the supernatant of lung homogenate increased to half of the amount recovered from the lungs at later sampling times. The dose that was initially deposited in the lungs was halved after five and 11 hours for the full-length antibody and the F(ab')₂ fragment, respectively. However, the amounts of PEG40-F(ab')₂ were only halved after 33 hours (**Fig. 1D****)**.

To assess the potential of 40-kDa PEG in prolonging the presence of other antibody fragments within the lungs in general, we delivered an anti-IL-13 Fab' fragment PEGylated with a two-armed PEG chain of 40 kDa (PEG40-Fab') and the unconjugated Fab' to the lungs in mice. We then measured the protein content in the respiratory tract post-delivery. The unconjugated fragment (47 kDa) and the PEG40-Fab' were fully cleared from nasal cavities in less than four hours (**Fig. 2A**)**.** The unmodified fragment was mostly cleared from the lungs within 24 hours (**Figs. 2B & C**)**,** as was anti-IL-17A F(ab')₂. The PEG40-Fab' appeared to remain within the lungs (BAL and supernatant of lung homogenate) longer than the unmodified Fab'. Specifically, 74% of the dose of PEGylated fragment that was initially deposited was still present after 24 hours, whereas only 32% of the unmodified fragment remained during the same period. After 48 hours, 40% of the PEG40-Fab' and 10% of the Fab' fragments were still present (**Fig. 2D**). Two-thirds of the PEGylated fragment was found in BAL over the first day after delivery, and one-third was found in the supernatant of lung homogenate. Forty-eight hours post-delivery, the fraction in BAL decreased to one-third and the fraction in the supernatant of lung homogenate increased to two-thirds (**Figs. 2B & C**). In comparison, the unconjugated fragment was mainly found in BAL over the first four hours after delivery and in the supernatant of lung homogenate 24 hours and 48 hours post-delivery. The dose that was initially deposited in the lungs was halved after four and 40 hours for the Fab' and PEG40-Fab', respectively (**Fig. 2D**).

### Therapeutic Efficacy of Antibody Constructs in a Murine Model of Allergen-induced Lung Inflammation

To investigate the potential therapeutic efficacy of the anti-IL-17A F(ab')₂ fragment conjugated to a two-armed 40-kDa PEG in an experimental model of lung inflammation, we administered the full-length anti-IL-17A antibody, the unconjugated anti-IL-17A F(ab')₂ fragment, the anti-IL-17A PEG40-F(ab')₂ and a control IgG to the respiratory tract of HDM-challenged mice. Upon allergen exposure, the mice treated with the PEG40-F(ab')₂ displayed lower eosinophilic infiltration, less peribronchial inflammation, strongly reduced glandular hyperplasia and decreased peribronchial smooth muscle cell layer thickness compared to the other HDM-challenged mice (**Figs. 3A-E**)**.** Mice treated with the PEG40-F(ab')₂ displayed a decreased bronchial reactivity after allergen exposure compared to HDM-challenged mice treated with PBS, the unconjugated anti-IL-17A F(ab')₂ fragment and the control IgG. The mice treated with the PEG40-F(ab')₂ exhibited similar bronchial reactivity to that displayed by mice treated with the full-length anti-IL-17A antibody (**Fig. 3F**). The contents of IL-17, IL-13 and CCL-11 in lung protein extracts after allergen exposure were decreased in mice treated with the PEG40-F(ab')₂ as compared to the other HDM-challenged mice (**Figs. 7A-C**)**.**

### Mechanisms Involved in the Increased Residence Time of PEGylated Antibody Fragments in the Lungs

Different mechanisms might explain the prolonged residency of antibody fragments within the lungs following PEGylation. These mechanisms include increased protein size, increased protein resistance against proteolysis, increased adhesiveness to mucus, decreased mucus clearance and protection against endocytosis by alveolar macrophages. We carried out additional experiments to investigate these different hypotheses.

To distinguish between the impact of molecular size and mucoadhesion on protein pulmonary fate, we compared the local pharmacokinetics of 40-kDa PEG (PEG40), 70-kDa dextran (dextran70) and anti-IL-17A F(ab')₂ in the lungs of mice (**Fig. 4A**). These three macromolecules have a similar hydrodynamic size of approximately 10 nm. Although the protein is not mucoadhesive, both polymers are mucoadhesive. However, linear and highly flexible PEGs are significantly more mucoadhesive than highly branched dextrans. Dextran70 was as rapidly cleared from the lungs as the F(ab')₂ fragment. However, PEG40 was cleared very slowly, and the lung-deposited dose was only reduced by 1.6-fold after two days **(****Fig. 4A****).** This finding suggests that the adhesiveness of PEG to mucus slowed down pulmonary clearance and that molecular size was not a dominant factor in clearance.

Additional information can be drawn from the macromolecule pharmacokinetics within the lungs by considering the metabolic stability of the various compounds **(****Fig. 4A****).** Both PEG and dextran are not degraded over 48 hours, whereas proteins can be degraded by proteases over a similar time period. The non-biodegradable dextran was eliminated from the lungs as quickly as the less stable protein, suggesting that the prolonged residency of PEGylated proteins within the lungs did not mainly result from the increased metabolic stability provided by PEG.

To assess whether PEG alone had an impact on protein residency within the lungs, we studied the fate of the anti-IL-13 Fab' in the presence and in the absence of PEG40. The protein showed the same local pharmacokinetics with or without the addition of PEG40, whereas the conjugate PEG40-Fab' appeared to remain longer in the lungs **(****Fig. 4B****).** Therefore, the presence of PEG alone does not prolong the protein half-life within the lungs, and the Fab' needs to be conjugated to PEG to observe an increase in residence time.

Finally, we visualized protein fate in the pulmonary tissue in mice using confocal laser scanning microscopy, assessing in particular whether protein uptake by alveolar macrophages could be hindered by PEG. Immediately after delivery, the solutions of both Alexa488-labeled PEG40-Fab' and Alexa568-labeled Fab' appeared to fill the airspaces of the murine lungs. Four hours post-delivery, Alexa568-labeled Fab' appeared as a ring on the surface of the alveolar epithelium, and 24 hours post-delivery, most of the non-PEGylated protein had been cleared from the tissue **(****Fig. 4C****).** In contrast, Alexa488-labeled PEG40-Fab' filled entire lung airspaces for at least the first 24 hours and, thus, remained in the pulmonary tissue longer than Alexa568-labeled Fab'. This observation is in agreement with the pharmacokinetic results **(****Fig. 4C****;** **Fig. 2****).** Alexa568-labeled Fab' and Alexa488-labeled PEG40-Fab' were both taken up by alveolar macrophages, indicating that PEGylation apparently did not prevent the endocytosis of the protein by local phagocytes (arrows in **Fig. 4C****;** **Fig. 8****).**

In conclusion, these results show that the coupling of large PEG chains to antibody fragments sustains their presence within the lungs for more than 2 days, whereas unconjugated counterparts are mostly cleared from the lungs within one day.

## Claims

1. A compound comprising one or more PEG moieties, wherein said compound is a therapeutic agent active for treating a respiratory disease, wherein the PEG moiety has a molecular weight of more than 12 kDa, provided that said therapeutic agent is not an anti-IL17 antibody or a fragment thereof.

2. The compound according to claim **1,** wherein said compound is selected from peptides, polypeptides and proteins, preferably is selected from the group comprising inhibitors of cyotkines, inhibitors of adhesion molecules, inhibitors of proteases, antibodies and antibody fragments, cytokines, decoy cytokines, cytokine receptors, deoxyribonucleases, immunosuppressant drugs.

3. The compound according to claim **1** or **2,** wherein said therapeutic agent is dornase alpha.

4. The compound according to anyone of claims **1** to **3,** wherein said respiratory disease is selected from inflammatory lung diseases, obstructive lung diseases, restrictive lung diseases, respiratory tract infections, malignant tumors, benign tumors, pleural cavity diseases, pulmonary vascular diseases, emphysema and pulmonary hyperplasia, preferably said respiratory disease is asthma or cystic fibrosis.

5. The compound according to any one of claims **1** to **4,** wherein the PEG moiety has a molecular weight of at least 20 kDa, preferably of at least 40 kDa.

6. The compound according to any one of claims **1** to **5,** wherein the PEG moiety is branched or forked.

7. A PEGylated therapeutic agent for use in treating a respiratory disease, wherein said PEGylated therapeutic agent is to be administered by respiratory administration.

8. The PEGylated therapeutic agent according to claim 7, wherein said compound is selected from peptides, polypeptides and proteins, preferably is selected from the group comprising inhibitors of cyotkines, inhibitors of adhesion molecules, inhibitors of proteases, antibodies and antibody fragments, cytokines, decoy cytokines, cytokine receptors, deoxyribonucleases, immunosuppressant drugs.

9. The PEGylated therapeutic agent according to claim **7** or **8,** wherein said respiratory disease is selected from inflammatory lung diseases, obstructive lung diseases, restrictive lung diseases, respiratory tract infections, malignant tumors, benign tumors, pleural cavity diseases, pulmonary vascular diseases, emphysema and pulmonary hyperplasia, preferably said respiratory disease is asthma or cystic fibrosis.

10. The PEGylated therapeutic agent according to any one of claims **7** to **9,** wherein the PEG moiety of the PEGylated therapeutic agent has a molecular weight of at least 12 kDa, preferably of at least 20 kDa, more preferably of at least 40 kDa.

11. The PEGylated therapeutic agent according to any one of claims **7** to **10,** wherein the PEG moiety of the PEGylated therapeutic agent is linear, branched or forked.

12. A method for enhancing the bioavailability of a compound to be administered by respiratory administration, preferably by inhalation, wherein said method comprises attaching one or more PEG moieties on said compound.

13. A method for reducing the pulmonary clearance of a compound, wherein said method comprises attaching one or more PEG moieties to the compound.

14. A method for enhancing the pulmonary residency of a compound, wherein said method comprises attaching one or more PEG moieties to the compound.

15. The method according to claim **14,** wherein the pulmonary residency of the pulmonary compound is of at least 24 hours, preferably of at least 36 or 48 hours.
